# EUROPEAN PATENT APPLICATION

(11) **EP 4 649 859 A1**
(43) Date of publication of application: **19.11.2025**
(21) Application number: 25172344.1
(22) Date of filing: 24.04.2025
(51) Int. Cl.: A45D 34/04, A61M 35/00, A45D 40/26

(54) **SUBSTANCE APPLICATOR**

(30) Priority: 10.05.2024 IT 202400010549
(71) Applicant: Myc Packaging Technology (Suzhou) Co., Ltd, Suzhou, Jiangsu Province 215011 (CN)
(72) Inventor: BOSE, Rahul, New Delhi (IN); Srivastava, Smita, 110017 New Delhi (IN); Kohli, Manisha, 110048 New Delhi (IN)
(74) Representative: Lunati & Mazzoni S.r.L.

(57) **Abstract**

The present invention provides a substance applicator that includes an applicator body made of a deformable elastomeric material and comprising a distal end of the applicator and one or more openings provided at the distal end of the applicator. A planar drapable structure made of a flexible and permeable material, the planar drapable structure being configured to be positioned on the distal end of the applicator, and the planar drapable structure being further configured to be in contact with the skin of a user. The substance applicator also includes an outer sleeve body comprising a distal end of sleeve, and the distal end of sleeve comprising a sleeve opening. Furthermore, the planar drapable structure in bending at the distal end of the applicator is configured to protrude at least partially outward from the sleeve opening.

## Description

### TECHNICAL FIELD

The present invention generally relates to devices used for the application of substance on outer surfaces of a user's body. More specifically, the present invention relates to substance applicators that are used for the application of substance employed for cosmetic purposes such as lipsticks, deodorants or nail polishes, or for the application of other forms of chemical substances such as pharmaceutical compositions for antiseptic use.

### PRIOR ART

The human skin is the largest organ of the human body and provides an outer covering to the human body, thereby protecting the internal tissues and organs from infections, from water loss in the form of dehydration, and from harmful radiations including ultraviolet light and the like. Furthermore, the skin is capable of generating Vitamin D upon absorption of sunlight. Therefore, proper skin care is required for maintaining its good health. External environmental agents and internal body conditions may affect the skin, leading to skin disorders or altered skin conditions such as wrinkles, sunspots, acne, discolorations, scars, pigmentation, rosacea, actinic keratosis, and undesired hair growth, to name a few. Since skin characteristics are also a measure of the aesthetic attractiveness of individuals, most people prefer non-invasive or minimally invasive therapies for the treatment of skin disorders. Furthermore, individuals also prefer to maintain the aesthetic attractiveness of the skin through the application of cosmetic substances. Such therapies and cosmetic substances are generally applied using substance applicators.

Substance applicators, also commonly referred to as velvet applicators in the context of the cosmetic industry, come in various shapes and sizes. For example, self-tanning applicators are in the form of a glove and help ensure a streak-free and even application of a self-tanning material. Similarly, makeup applicators may be used to apply make-up such as foundation, blush, and bronzer. Lipstick applicators are specifically designed to apply lipsticks. For pharmaceutical applications, various forms of applicators are used. For example, swabs, sponge sticks, and gauze-like pads pre-saturated with antiseptic solutions are often used to treat skin infections and to medicate wounds. Furthermore, applicator bottles that include a reservoir filled with an antiseptic solution and an applicator tip or an applicator brush may also be used for antiseptic use.

However, as may be seen from the above description, different applicator constructions apply to different types of substances and different types of applications. Few solutions in the prior art may serve more than one or two purposes. Therefore, it becomes necessary for the user to purchase most of the different devices described above if they expect to encounter many, if not all, of the situations mentioned above, leading to higher costs and material waste. Therefore, there is a need in the art for a substance applicator that does not exhibit the above shortcomings.

### OBJECTS OF THE INVENTION

Some of the objects of the present invention are as follows:
An object of the present invention is to provide a substance applicator that may be used both for cosmetic and medicinal purposes.

Another object of the present invention is to provide a substance applicator that is capable of being attached to different reservoirs containing different types of substances and of applying that substance to the outer surfaces of a user's body. Another object of the present invention is to provide a substance applicator that utilizes an antibacterial surface for the application of the substance stored in the reservoir.

Another object of the present invention is to provide a substance applicator that is simple in construction and convenient for assembly, cleaning, maintenance, reuse, and recycling.

Another object of the present invention is to provide a substance applicator that may be used for different types of substances such as facial serums, skin moisturizers, oils, and pharmaceutical compositions, etc., which may be available in the form of liquids, gels, emulsions, aerosols, and solutions, etc., and are used to treat related skin conditions and/or enhance the overall aesthetic attractiveness and general health of a user's skin.

### SUMMARY OF THE INVENTION

According to a first aspect of the present invention, a substance applicator is provided. The substance applicator includes a deformable applicator body comprising a distal end of the applicator and one or more openings provided at the distal end of the applicator, the one or more openings being configured to dispense a substance. Furthermore, the substance applicator includes an outer sleeve body configured to connect the applicator body, the outer sleeve body including a distal end of sleeve, and the distal end of sleeve including a sleeve opening. Furthermore, the distal end of the applicator is configured to protrude at least partially outward from the sleeve opening.

In an embodiment of the invention, the substance applicator further includes a planar drapable structure made of a flexible and permeable material, the planar drapable structure being configured to be positioned over the distal end of the applicator, and the planar drapable structure being further configured to be in contact with the skin of a user. The outer sleeve body is configured to connect the applicator body and the planar drapable structure so as to maintain the flexibility of the connected planar drapable structure. The connected planar drapable structure is configured to protrude at least partially outward from the sleeve opening.

In an embodiment of the invention, when assembled, the planar drapable structure is further configured to be encapsulated between the outer sleeve body and the applicator body and to undergo deformation and conform to a shape of the applicator body.

In an embodiment of the invention, the planar drapable structure is made of a material selected from a fabric, a flexible mesh, permeable material infused with an antibacterial material such as silver, or any other planar permeable material.

In an embodiment of the invention, the substance applicator further includes a nozzle body configured to be attached to a substance reservoir, the nozzle body being configured to receive within it the substance stored in the substance reservoir. Furthermore, the nozzle body includes a proximal end of nozzle configured to be attached to an outlet of the substance reservoir, and a distal end of nozzle configured to release the received substance.

In an embodiment of the invention, the proximal nozzle end includes a nozzle collar with one or more second nozzle coupling members, the one or more second nozzle coupling members being configured to engage one or more complementary reservoir coupling members of the substance reservoir.

In an embodiment of the invention, the distal end of the applicator includes a convex beveled surface on which the one or more openings are provided.

In an embodiment of the invention, at least the distal end of the applicator is made of a deformable material and is configured to be inwardly compressed.

In an embodiment of the invention, at least the distal end of the applicator of the applicator body and the planar drapable structure positioned on the distal end of the applicator are configured to be inwardly compressed.

In an embodiment of the invention, the hardness of the deformable material of the applicator body varies between 50 and 60 Shore A.

In an embodiment of the invention, the applicator body includes an applicator outer surface in fluid communication with an inner surface of applicator through one or more substance applicator channels, the applicator outer surface comprising the one or more openings, the applicator inner surface defining an inner cavity of applicator, and the one or more substance applicator channels terminating at respective said one or more openings.

In an embodiment of the invention, the inner applicator surface is configured to engage one or more first nozzle coupling members provided on a nozzle outer surface of the nozzle body.

In an embodiment of the invention, the applicator body includes an applicator collar and one or more applicator coupling members located on an applicator outer surface, and between the distal end of the applicator and a proximal end of the applicator to the substance reservoir, the applicator collar being configured to be positioned over the outer sleeve body, and the one or more applicator coupling members being configured to engage one or more complementary sleeve coupling members of the outer sleeve body.

In an embodiment of the invention, the substance is selected from a group consisting of liquids, gels, aerosols, pseudoplastic fluids, and combinations thereof. According to a second aspect of the present invention, a substance applicator is provided. The substance applicator includes a nozzle body configured to be attached to a substance reservoir, the nozzle body being configured to receive within it a substance stored in the substance reservoir. Furthermore, the nozzle body includes a proximal end of the nozzle configured to be attached to an outlet of the substance reservoir, and a distal end of the nozzle configured to release the received substance. The substance applicator further includes an applicator body configured to connect the nozzle body, the applicator body including a distal end of the applicator, and one or more openings provided at the distal end of the applicator, the one or more openings being configured to dispense the substance released from the nozzle body. Furthermore, the substance applicator includes a planar drapable structure made of a flexible and permeable material, the planar drapable structure being configured to be positioned over the distal end of the applicator, and the planar drapable structure being further configured to be in contact with the skin of a user to apply the substance. The substance applicator also includes an outer sleeve body configured to connect the applicator body, the outer sleeve body including a distal end of sleeve, and the distal end of sleeve including a sleeve opening. Preferably, the outer sleeve body is configured to connect the deformable applicator body and the planar drapable structure so as to maintain the flexibility of the connected planar drapable structure. The distal end of the applicator is configured to protrude at least partially outward from the sleeve opening. Furthermore, the connected planar drapable structure is configured to protrude at least partially outward from the sleeve opening. Even when assembled, the planar drapable structure is further configured to be encapsulated between the outer sleeve body and the applicator body and to undergo deformation and conform to the shape of the applicator body.

In the context of the description, the terms "polymer" or "plastic" refer to a material composed of long chains of organic molecules (of eight or more organic molecules) that include, but are not limited to, carbon, nitrogen, oxygen, and hydrogen as their constituent elements. The term polymer is intended to include both naturally occurring polymers such as wool, and synthetic polymers such as polyethylene and nylon.

In the context of the description, the terms "distal" and "proximal" are used with claimed and/or described elements relative to the respective positions of the claimed and/or described elements with respect to the substance reservoir.

### BRIEF DESCRIPTION OF THE ACCOMPANYING DRAWINGS

The accompanying drawings illustrate the best mode for carrying out the invention as presently contemplated and set forth below. The present invention may be better understood by a careful review of the following detailed description of preferred embodiments taken in conjunction with the accompanying drawings in which like reference letters and numerals indicate corresponding parts in the various figures in the accompanying drawings, and wherein:
**Figure 1** illustrates a perspective view of a substance applicator, according to an embodiment of the invention;
**Figure 2A** illustrates an exploded view of the substance applicator, according to an embodiment of the invention;
**Figure 2B** illustrates a sectional view of the substance applicator of Fig. 2A in the exploded state;
**Figure 3** illustrates a sectional view of the substance applicator of Fig. 2A in the assembled state;
**Figure 4** illustrates an exploded view showing the substance applicator, a cap, and a substance reservoir, according to an embodiment of the present invention; and
**Figure 5** illustrates a user using the substance applicator attached to the substance reservoir, according to an embodiment of the present invention.

### DETAILED DESCRIPTION

The embodiments of the present description of the invention will be described in more detail below with reference to the accompanying drawings in which similar numerals represent similar elements throughout the figures, and in which exemplary embodiments are shown.

The detailed description and the accompanying drawings illustrate specific embodiments by which the description may be implemented. These embodiments are described in detail to enable those skilled in the art to implement the invention illustrated in the description. It is understood that other embodiments may be used, and further modifications may be made, without departing from the spirit or scope of the present description. The following detailed description is therefore not to be taken in a limiting sense, and the scope of the present description of the invention is defined by the appended claims. Embodiments of the claims may, however, be implemented in many different forms and should not be construed as limited to the embodiments set forth herein.

The embodiments of the present invention provide a substance applicator. The substance applicator includes an applicator body and an outer sleeve body. Preferably, the applicator body is a deformable applicator body. Furthermore, the substance applicator includes a planar drapable structure. The planar drapable structure is flexible and permeable. The planar drapable structure is made of a material selected from a fabric, a flexible mesh, permeable material infused with an antibacterial material such as silver, or any other planar permeable material. Furthermore, the substance applicator includes a nozzle body. The nozzle body is configured to be attached to a substance reservoir and to receive within it the substance to be applied to the skin of a user. The applicator body connects the nozzle body, and the nozzle body dispenses the substance from the substance reservoir to the applicator body. The applicator body includes an inner cavity of the applicator defined by an inner surface of the applicator. The substance received from the nozzle body is then dispensed outward from one or more openings provided in an applicator outer surface at the distal end of the applicator of the applicator body. Furthermore, the applicator body is made of a deformable elastomeric material. At least, the distal end of the applicator is made of a deformable material such as, for example, elastomers; the distal end of the applicator may, in detail, have a hardness value between 20 and 60 Shore A. In this sense, at least the distal end of the applicator may be made of soft urethanes used for particle impact applications. Furthermore, the planar drapable structure and the distal end of the applicator are configured to be inwardly compressible toward the inner cavity of applicator.

The applicator outer surface is in fluid communication with the applicator inner surface and the inner cavity of applicator, through one or more substance applicator channels that terminate at respective said one or more openings. The outer sleeve body connects the applicator body and allows at least a part of the distal end of the applicator to protrude outward through a sleeve opening provided at the distal end of the sleeve of the outer sleeve body. Preferably, the outer sleeve body is configured to connect the deformable applicator body and the planar drapable structure so as to maintain the flexibility of the connected planar drapable structure, and the connected planar drapable structure is configured to protrude at least partially outward from the sleeve opening. The planar drapable structure in this sense may be positioned on the distal end of the applicator and is configured to be in contact with the skin of the user to apply the substance. The outer sleeve body connecting the applicator body ensures that the drapable structure is deformed and encapsulated between the outer sleeve body and the applicator body, and the planar drapable structure conforms to the shape of the applicator body.

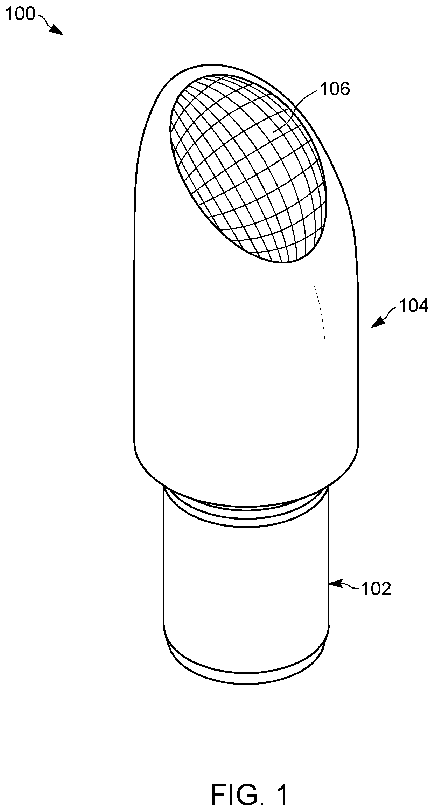

Various embodiments of the present invention will now be described in detail with reference to Figs. 1 to 5.

Fig. 1 illustrates a perspective view of a substance applicator **100,** according to an embodiment of the invention. The substance applicator 100 includes an applicator body **102,** an outer sleeve body 104, and a planar drapable structure 106. The applicator body 102 is a deformable applicator body and is made of a deformable elastomeric material, preferably of a deformable elastomeric material. Some examples of elastomers include natural rubber, polybutadiene, silicone, neoprene, polyurethanes, styrene-butadiene block copolymers, polyisoprene, ethylene propylene rubber, ethylene propylene diene rubber, fluoroelastomers, nitrile rubbers, etc. The outer sleeve body 104 may be made of a rigid or flexible polymeric or plastic material such as polycarbonate (PC), polymethyl methacrylate (PMMA), polyvinyl chloride (PVC), acrylonitrile butadiene styrene (ABS), high-density polyethylene (HDPE), nylon or polyamide (PA), polytetrafluoroethylene (PTFE), polypropylene (PP), and the like. Furthermore, in various embodiments of the invention, the hardness of the deformable material of the applicator body 102 varies between 50 and 60 Shore A.

Furthermore, the planar drapable structure 106 is permeable and flexible. The planar drapable structure 106 is made of a material selected from a fabric, a flexible mesh, permeable material infused with an antibacterial material such as silver, or any other planar permeable material. Furthermore, in use, the planar drapable structure 106 is configured to be in contact with the skin of a user. Therefore, in various embodiments of the invention, the planar drapable structure 106 may be infused with an antibacterial material such as silver (Ag).

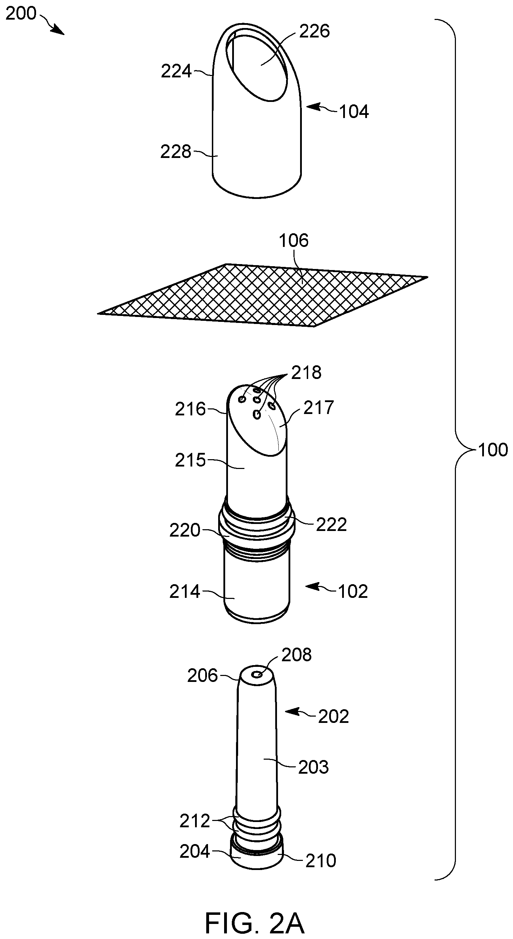

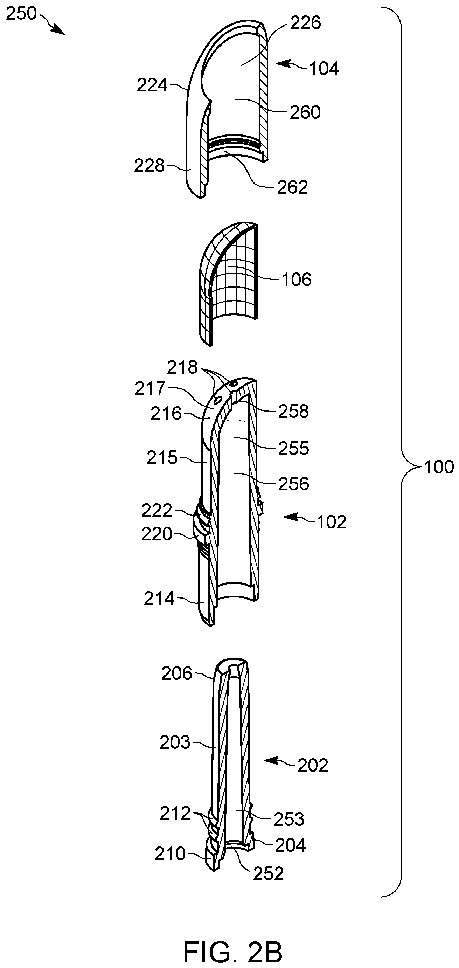

Fig. 2A illustrates an exploded view **200** of the substance applicator 100, according to an embodiment of the invention. Fig. 2B illustrates a sectional view of the substance applicator of Fig. 2A in the exploded state. With reference to Figs. 2A and 2B, the outer sleeve body 104 includes a distal end of sleeve **224** and a proximal end of the sleeve **228.** The distal end of sleeve 224 further includes a sleeve opening **226.** The applicator body 102 includes a distal end of the applicator **216** and a proximal end of the applicator **214.** Furthermore, the applicator body 102 includes an applicator outer surface **215** and an inner surface of applicator **255.** The applicator inner surface 255 defines an inner cavity of applicator **256.** The applicator inner surface 255 and hence the inner cavity of applicator 256 are in fluid communication with the applicator outer surface 215 through one or more substance applicator channels **258.**

The one or more substance applicator channels 258 terminate at respective said one or more openings **218** provided in the applicator outer surface 215 at the distal end of the applicator 216. Furthermore, the distal end of the applicator 216 is provided with a convex beveled surface **217** on which the one or more openings 218 are provided. The convex beveled surface 217 allows improved contact with the user's skin. In other embodiments, the surface 217 may not be convex. Furthermore, the distal end of the applicator 216 and the planar drapable structure 106 are configured to be inwardly compressed toward the inner cavity of applicator 256. The applicator outer surface 215 further includes an applicator collar 220 and one or more applicator coupling members **222** located between the proximal end of the applicator 214 and the distal end of the applicator 216. The outer sleeve body 104 is configured to connect the deformable applicator body 102 and the planar drapable structure 106 so as to maintain the flexibility of the connected planar drapable structure 106. In this sense, the outer sleeve body 104 is configured to be positioned over the applicator collar 220 and the one or more applicator coupling members 222 are configured to engage one or more complementary sleeve coupling members **262** provided on an inner surface of sleeve **260** of the outer sleeve body 104. Furthermore, the distal end of the applicator 216 is configured to protrude at least partially outward from the sleeve opening 226. The connected planar drapable structure 106 and the distal end of the applicator 216 are configured to protrude at least partially outward from the sleeve opening 226. The planar drapable structure 106 is configured to be positioned on the distal end of the applicator 216 and encapsulated between the outer sleeve body 104 and the applicator body 102. Even during the assembly of the substance applicator 100, the planar drapable structure 106 is configured to undergo deformation and to conform to the shape of the applicator body 102. The distal end of the applicator 216 of the applicator body 102 and the planar drapable structure 106 positioned on the distal end of the applicator 216 are configured to be inwardly compressed. Therefore, the outer sleeve body 104 is configured to connect the applicator body 102 and the planar drapable structure 106 so as to maintain the planar drapable structure 106 in bending, particularly conforming to the applicator body 102, and connected to the applicator body 102.

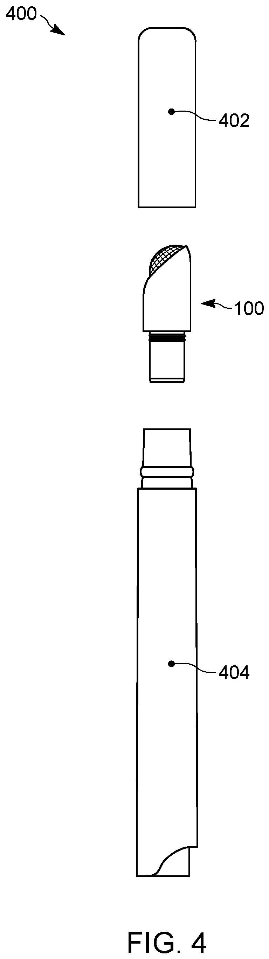

The substance applicator 100 also includes a nozzle body 202. The nozzle body 202 includes a nozzle proximal end 204 configured to be attached to an outlet of a substance reservoir (see Fig. 4). Furthermore, the nozzle body 202 includes a nozzle distal end 206 provided with a nozzle opening 208. The applicator body 102 is configured to connect the nozzle body 202, such that the distal nozzle end 206 is inserted into the inner cavity of applicator 256. In this sense, in various embodiments of the invention, the nozzle body 202 may include a tapered profile between the proximal nozzle end 204 and the distal nozzle end 206. Furthermore, the nozzle body 202 includes one or more first nozzle coupling members 212 provided on an outer nozzle surface **203** of the nozzle body 202. In various embodiments of the invention, the one or more first nozzle coupling members 212 may be embodied as rigid projections. Alternatively, the one or more first nozzle coupling members 212 may also be formed as threaded portions or projections or recesses with snap-fit arrangements.

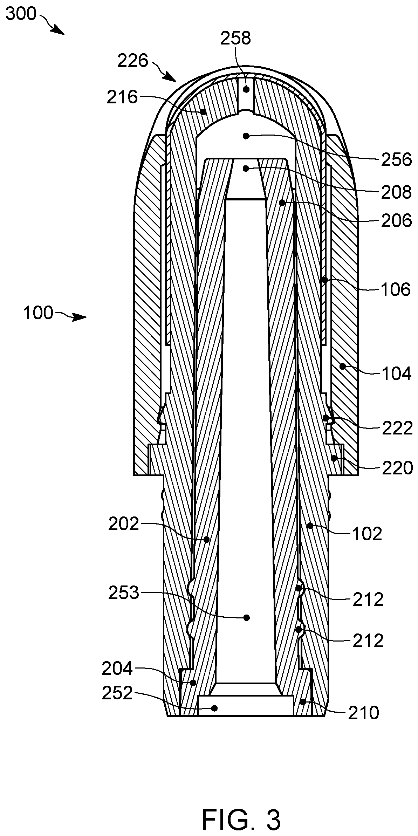

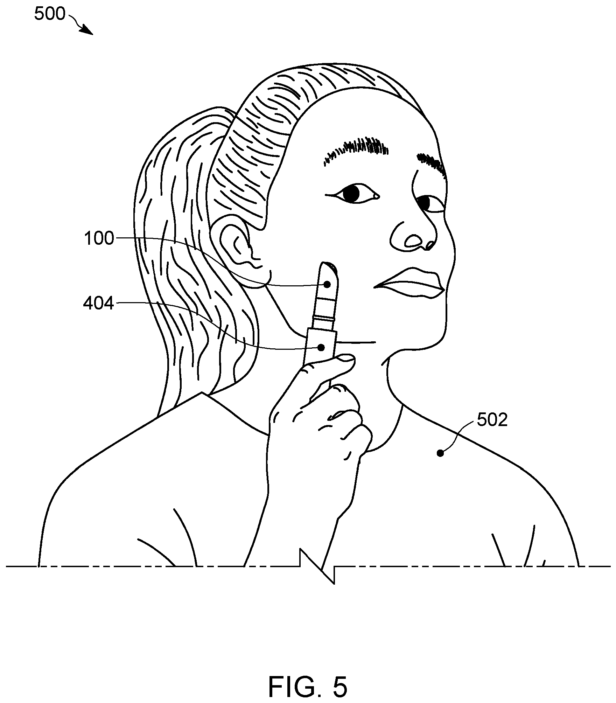

The one or more first nozzle coupling members 212 are configured to engage the applicator inner surface 255 of the applicator body 102. The engagement may be accomplished by plastic deformation of the applicator inner surface 255 or the applicator inner surface 255 may be provided with complementary grooves that may then engage the one or more first nozzle coupling members 212. Furthermore, the nozzle body 202 is configured to be attached to a substance reservoir (see Fig. 4). The proximal nozzle end 204 includes a nozzle collar 210 with one or more second nozzle coupling members 252 provided on an inner nozzle surface 253. The one or more second nozzle coupling members 252 may be formed as internal threads configured for engagement with complementary reservoir coupling members (not shown) of the substance reservoir. For example, the complementary reservoir coupling members may be formed as external threads of the substance reservoir. Fig. 3 illustrates a sectional view **300** of the substance applicator 100 of Fig. 2A in the assembled state. The nozzle body 202 is configured to be attached to the substance reservoir at the proximal nozzle end 204. In this sense, the one or more second nozzle coupling members 252 of the nozzle collar 210 may be formed as internal threads that may be configured for engagement with complementary reservoir coupling members (not shown), for example, formed as external threads of the substance reservoir. Fig. 4 illustrates an exploded view showing the substance applicator 100, a cap 402, and a substance reservoir 404, according to an embodiment of the present invention. Fig. 5 illustrates a user **502** using the substance applicator 100 attached to the substance reservoir 404, according to an embodiment of the present invention.

With reference to Figs. 3, 4, and 5, the nozzle body 202 is configured to receive within it a substance stored within the substance reservoir 404. The substance may be selected from a group consisting of liquids, gels, aerosols, pseudoplastic fluids, and combinations thereof. Furthermore, the distal nozzle end 206 is configured to release the received substance from the nozzle opening 208 and into the inner cavity of applicator 256. The one or more openings 218 are configured to receive the substance from the inner cavity of applicator 256 through the one or more substance applicator channels 258. Furthermore, the one or more openings 218 are configured to dispense the substance released from the nozzle body 202. The planar drapable structure 106 is configured to be in contact with the user's skin to apply the substance. In this sense, the distal end of the applicator 216 and the planar drapable structure 106 are configured to protrude at least partially outward from the sleeve opening 226.

Various modifications to these embodiments will be apparent to those skilled in the art from the description and the accompanying drawings. The principles associated with the various embodiments described herein may be applied to other embodiments. Therefore, the description is not intended to be limited to the embodiments shown with the accompanying drawings but is to provide the broadest scope consistent with the principles and novel features described or suggested herein. Accordingly, the invention intends to encompass all such alternatives, modifications, and variations that fall within the scope of the present invention and the appended claims.

## Claims

1. Applicator (1) of substance comprising:
- an elastomeric deformable applicator body (102) including:
- a distal end of the applicator (216), and
- one or more openings (218) provided at said distal end of the applicator (216) configured to deliver a substance;
- a planar drapable structure (106), flexible and permeable configured to be positioned over said distal end of the applicator (216), and to be in contact with a skin of a user (502) to apply said substance;
and **characterised by** comprising further
- an outer sleeve body (104) configured to connect said applicator body (102) and said planar drapeable structure (106) so as to maintain said planar drapeable structure (106) in flexion and connected to said applicator body (102), and comprising a distal end of sleeve (224) including a sleeve opening (226);
and by **the fact** that
- said one or more openings (218) are configured to dispense said substance toward said planar drapable structure (106), and
- said planar drapable structure (106) in bending is configured to protrude at least partially outward from said sleeve opening (226).

2. Applicator (1) of substance according to claim 1, wherein, when said applicator (1) is assembled, said planar drapable structure (106) is further configured to be encapsulated between said outer sleeve body (104) and said applicator body (102), and undergo deformation and conform to the shape of said applicator body (102).

3. Applicator (1) of substance according to any previous claim, wherein the planar drapable structure (106) is made of a material selected from a fabric, flexible mesh, material infused with an antibacterial material such as silver, or any other planar permeable material.

4. Applicator (1) of substance according to any of the previous claims, wherein said distal end of said applicator (216) of said applicator body (102) comprises a convex beveled surface (217) on which said one or more openings (218) are provided.

5. Applicator (1) of substance according to any of the previous claims, wherein at least said distal applicator end (216) of said applicator body (102) and said planar drapable structure (106) positioned on said distal applicator end (216) are configured to be inwardly compressed.

6. Applicator (1) of substance according to any previous claim, wherein the hardness of elastomeric material varies between 20 Shore A and 60 Shore A.

7. Applicator (1) of substance according to any of the previous claims, wherein said applicator body (102) comprises an applicator outer surface (215) in fluid communication with an inner surface of applicator (255) through said one or more substance applicator channels (258), said applicator outer surface (215) includes said one or more openings (218), said inner surface of applicator (255) defines an inner cavity of applicator (256), and said one or more channels of substance applicator (258) terminate at respective said one or more openings (218).

8. Applicator (1) of substance according to the previous claim, wherein said inner applicator surface (255) is configured to engage one or more first nozzle coupling members (212) provided on an outer nozzle surface (203) of a nozzle body (202).

9. Applicator (1) of substance according to any preceding claim, further comprising a nozzle body (202) configured to be attached to a substance reservoir (404) and configured to receive within it said substance stored in said substance reservoir (404), and wherein said nozzle body (202) comprises a proximal end of nozzle (204) configured to be attached to an outlet of said substance reservoir (404), and a distal end of nozzle (206) configured to release said received substance.

10. Applicator (1) of substance according to the previous claim, wherein said proximal nozzle end (204) comprises a nozzle collar (210) having one or more second nozzle coupling members (212) configured to engage one or more complementary reservoir coupling members of said substance reservoir (404).

11. Applicator (1) of substance according to any of the previous claims, wherein said applicator body (102) comprises an applicator collar (220) and one or more applicator coupling members (222) located on an applicator outer surface (215), and between a distal end of said applicator (216) and a proximal end of said applicator (214), said applicator collar being configured to position on said outer sleeve body (104), and said one or more applicator coupling members (222) being configured to engage one or more complementary sleeve coupling members (262) of said outer sleeve body (104).

12. Applicator (1) of substance according to any preceding claim, wherein said substance is selected from a group consisting of liquids, gels, aerosols, pseudoplastic fluids, and combinations thereof.

13. Applicator (1) of substance according to any of the previous claims wherein at least said distal end of the applicator (216) and said planar drapable structure (106) positioned on said distal end of the applicator (216) are configured to be inwardly compressed.

14. Applicator (1) of substance according to any one of claims 9-13, wherein said applicator body (102) is configured to accommodate said nozzle body (202).
